# EUROPEAN PATENT SPECIFICATION

(11) **EP 3 027 169 B1**
(45) Date of publication and mention of the grant of the patent: **22.03.2017**
(21) Application number: 14747872.1
(22) Date of filing: 22.07.2014
(51) Int. Cl.: A61K 8/44, A61K 8/46, A61K 8/92, A61K 8/02, A61K 8/04, A61Q 1/14, A61Q 9/02, A61Q 19/10, A61K 8/31

(54) **FOAMABLE PERSONAL CARE COMPOSITION COMPRISING A CONTINUOUS OIL PHASE**
SCHÄUMBARE KOSMETISCHE ZUSAMMENSETZUNG MIT KONTINUIERLICHER ÖLPHASE
COMPOSITION DE SOIN PERSONNEL MOUSSANTE COMPRENANT UNE PHASE CONTINUE HUILEUSE

(30) Priority: 01.08.2013 US 201361861001 P
(43) Date of publication of application: 08.06.2016
(73) Proprietor: Unilever PLC, a company registered in England and Wales under company no. 41424, Greater London EC4Y 0DY (GB); Unilever N.V., 3013 AL Rotterdam (NL)
(72) Inventor: TSAUR, Liang, Sheng, Trumbull, Connecticut 06611 (US)
(74) Representative: Fijnvandraat, Arnoldus
(86) International application number: PCT/EP2014/065707
(87) International publication number: WO 2015/014667

(56) References cited:
- US-A- 4 026 825
- US-A- 4 093 745
- DATABASE WPI Week 201062 Thomson Scientific, London, GB; AN 2010-L46367 XP002731765, & JP 2010 195694 A (MIYOSHI KASEI YG) 9 September 2010 (2010-09-09)

## Description

### Field of the invention

This invention relates to foamable personal care compositions including, for example, cleansing compositions, make-up removers, shaving compositions, and other lather-off compositions for skin and/or hair treatment which compositions comprise a continuous oil phase stabilized with aggregates of dispersed solid surfactant particles.

### Background of the invention

Many foamable personal care compositions, are water-based fluids that include one or more solid cleansing surfactants. The compositions, commonly oil-in-water emulsions, typically require production techniques that employ one or more heating steps in which a surfactant-containing aqueous phase is heated to temperatures on the order of 50-90°C, followed by cooling and subsequent combination with an oil phase and other composition ingredients, e.g., volatiles. Heating and subsequent cooling can add to production costs and equipment requirements. Such requirements can also become time limiting steps during production. Additionally, creating concentrates with conventional water-based detergents can be problematic in that the reduced amount of water in a concentrate can give rise to products that are prone to gel formation, and as a result, too viscous to be easily applied or diluted.

Fluid compositions having a continuous oil phase offer potential advantages over conventional water-based compositions in one or more of the aforesaid areas, i.e., processing, concentrate formation, and so forth.

Certain oil-based personal care compositions have been suggested:
US 4,673,526 to Zabotto et al. describes an anhydrous skin cleansing composition containing an oil phase, an emulsifying agent and particulate water soluble polymeric abrasive particles.

US 8,063,005 to Kalidini discloses personal care formulations said to have simultaneous exfoliating, cleansing and moisturizing properties, which formulations comprise an oil phase and a natural surfactant derived from legumes and/or grains.

US 6,620,773 to Stork et al. discloses foaming oil preparations that include clear single-phase foaming oil formulations containing a mixture of oil and specific surfactants.

US 5,653,988 to Gerber et al. discloses substantially anhydrous clear shower oil composition containing up to 55% by weight of an amide of a fatty alcohol sulfate or an amide of a fatty alcohol ether sulfate.

US 6,524,594 to Santora et al. discloses a gelled oil skin cleansing composition comprising 3 to 10%, based on the total weight of the composition, of a gelling agent selected from dextrin myristate or a blend of at least two different polymers consisting of diblock or triblock copolymers. The patent further discloses that the gelled composition is "essentially water-free", therein understood to mean that based on the total weight thereof, the gelled composition contains no more than about 5 percent of water, and preferably comprises no more than about 3 percent of water.

US 2005/0158351A1 to Soliman et. al. discloses an anhydrous exfoliative foaming cleanser containing (1) 5 to 40wt.% of large exfoliative particles having a mean particle size 50 to 1200 microns to serve as scrubbing agent again the skin; (2) 0.5 to 15 wt.% anionic surfactants; (3) emollient oil and at least one oil gelling agent for phase stability. Exemplary of such gelling agents are silica, clays and organically modified clays and mixtures thereof.

Notwithstanding the foregoing, formulating continuous oil phase compositions with desirable phase stability has been problematic. When present as the disperse phase of an oil-based composition, solid surfactant particles tend to separate over time, forming what can be a dense layer of the particles at the bottom of the composition and an oil layer on the top. The tendency toward particle separation is increased with larger and/or heavier particles, i.e., particles having weight average particle size of greater than 50µm. Conventional routes to stabilization, e.g., the addition of viscosity modifiers and thickening agents, can interfere with surfactant release in use, impeding foam formation and lathering.

There remains a need for foamable personal care compositions, in particular fluid compositions, that can produced under conditions that minimize the need to employ heating and cooling and, if desired, that can be formulated over a broad range of solid cleansing surfactant concentrations, for example up to 65% by weight, more particularly 20 to 60 % by weight, based on the total weight of the composition. There remains a further need for foamable personal care compositions having a continuous oil phase and, more particularly, for storage stable compositions that, when foamed, have good lather properties.

### Summary of the invention

It has now been found that foamable fluid compositions comprising a continuous oil phase in which is dispersed aggregates of solid cleansing surfactant provide a product format that offers convenient preparation for personal care compositions, in particular, the ability to achieve, if desired, relatively high loadings of the surfactant in a composition that affords good phase stability, that is to say, the structure of the composition is less susceptible to collapsing and the solid cleansing surfactant is less susceptible to sedimentation. It has also been found that stabilization can be achieved using pre-aggregated solid surfactant particles and/or solid surfactant particles that are aggregated in situ by the addition of a small amount water, from 0.05 to 5% by weight, particularly, from 0.1 to 5 % by weight, more particularly from 0.2 to 4% by weight, based on the total weight of the composition. In one or more embodiments of particular interest the subject particles are aggregated in situ by the addition of water in an amount of from 0.2 to 3% by weight, based on the total weight of the composition.

Without wishing to be bound to theory, it is believed that the aggregated surfactant particles form a loose, stabilized structure in the oil phase. In addition to improving storage stability, this loose structure provides desirable surfactant release in use. In turn, the surfactant release properties aid in the production of creamy, thick foams.

Pursuant to the subject invention there is provided a foamable, fluid personal care composition that comprises a continuous oil phase comprising at least one carrier oil and dispersed aggregated particles of solid cleansing surfactant, wherein the composition comprises:
i. 35 to 90% by weight, preferably 40 to 80 % by weight, of carrier oil,
ii. 10 to 65% by weight, preferably from 20 to 60% by weight, of solid cleansing surfactant; and
iii. 0.05 to 5% by weight, preferably up to 4% by weight, more preferably up to 3% by weight, of water.

In use, the composition is diluted with water, dissolving and releasing the surfactant and generating lather.

### Detailed description of the invention

As used herein, the term "fluid" refers to a composition that at 1 atmosphere and 23°C has a viscosity in a range of from 300 to 50,000kgm⁻¹s⁻¹ (i.e., Pa.s) at 0.01 rps (reciprocal second) and 23°C. Owing to the way it is stabilized, the oil-continuous composition exhibits shear thinning rheology, i.e., viscosity decreases with increasing shear rate. The compositions of this invention typically have a viscosity at 0.01rps and 23°C that is at least 10 times higher than their viscosity at 1rps and 23°C. In one or more preferred embodiments the viscosity of the compositions at 1 rps and 23°C is in a range of 10 to 500 kgm⁻¹s⁻¹. Viscosity may be conveniently measured using a standard AR-G2 stress-controlled rheometer from Texas Instruments (or equivalent) and following the viscosity measurement procedure described in the Examples that follow. The viscosity range of interest encompasses a broad range of pourable and/or pumpable compositions including liquids, pastes, and creams.

The oil phase of the subject composition comprises one or more cosmetically acceptable carrier oils. Among the carrier oils suitable for use herein are natural and synthetic oils that are liquid at 1 atmosphere of pressure and 25°C. Included among such oils are hydrocarbon oils, ether oils, ester oils, fatty alcohols, and silicone oils. Among the suitable hydrocarbon oils are linear, branched and cyclic oils, non-limiting examples of which include, for example, liquid paraffin, squalene, squalane, mineral oil, low viscosity synthetic hydrocarbons such as, for example, polyalphaolefin sold by ExxonMobil under the trade name of PureSyn™ POA, polybutene, including oils available from INEOS under the trade name of PANALANE^{®} or INDOPOLE^{®}, and diethyl hexyl cyclohexane. Light (low viscosity) highly branched hydrocarbon oils are also suitable.

Among the suitable ester oils are, for example, mono- and polyfunctional esters such as cetyl octanoate, octyl isonanoate, myristyl lactate, cetyl lactate, cetyl ethyl hexanoate, isopropyl myristate, myristyl myristate, isopropyl palmitate, isopropy adipate, diisopropyl adipate, isopropyl isostearate, butyl stearate, decyl oleate, isodecyl oleate, glycerol monostearate, glycerol distearate, glycol tristearate, and glyceryl tri(2-ethylhexanoate); as well as modified ester oils such as, for example, alkoxylated esters including, for example PPG-3-benzyl ether myristate, and the like. Other suitable ester oils are triglycerides and modified triglycerides. These include vegetable oils such as, for example, jojoba, safflower, sunflower seed, palm kernel, soybean, castor, coconut, olive, rice-germ, sweet almond, rapeseed, wheat-germ, and grape seed oil, and the like. Synthetic triglycerides may also be employed. Among the modified triglycerides are materials such as, for example ethoxylated and maleated triglyceride oils. Among the triglyceride oils, materials of particular interest include C₈-C₁₈, and especially the C₈-C₁₂, fatty acid triglycerides. Proprietary ester blends such as those sold by Finetex under the trade name Finsolv^{®}, may also be employed herein. Another type of suitable ester is liquid polyester formed from the reaction of a dicarboxylic acid and a diol including, for example, the polyester marketed by ExxonMobil under the trade name PureSyn™ Ester.

Ether oils suitable for use herein include, for example, polyglycols (especially from polypropylene glycol, PPG, the latter preferably containing at least 3 mers, such as 3 to 20), of monohydric alcohols (the monohydric alcohol often containing between 3 and 20 carbons). As the molecular weight of the PPG increases, the chain length of the monohydric alcohol can decrease. For example, suitable ether oils can vary between a low molecular weight PPG with a long chain fatty alcohol (an example of such an oil being PPG-3 myristyl ether) and a lower alkyl ether of a higher molecular weight PPG (such an example of such an oil being PPG-14 butyl ether).

The alcohols suitable for use herein, include, for example, oleyl alcohol, isostearyl alcohol, hexyl decanol, 2-octyldodecanol (Eutanol^{®} G) and the like.

Among the suitable silicone oils are linear and cyclic polydimethyl siloxanes, as well as organofunctional silicones (alkyl and alkyl aryl), and hydroxyl- and amino- silicones.

The carrier oils may be used individually or as combinations of two or more from the same or different classes. Of particular interest as carrier oils are hydrocarbon oils (mineral oil in particular), triglyceride oils and silicone oil.

In one or more embodiments all or substantially all, that is to say up to 90% by weight, preferably up to 95% by weight, more preferably up to 97% by weight of the carrier oil is non-volatile. Preferably the non-volatile carrier oil has a vapor pressure less than 0.5 mmHg at 23°C. From a material handling perspective, the non-volatile oil preferably has a boiling point in excess of 100°C, more preferably in excess of 150°C.

In other embodiments it is contemplated that up to 30 wt.%, more particularly up to 15 wt.%, of the carrier oil is volatile oil. Replacement of a portion of the non-volatile carrier oil with a volatile carrier oil may provide or certain sensory advantages.

In the subject compositions carrier oil is present in an amount of from 35 to 90% by weight, preferably from 40 to 80% by weight, based on the total weight of the composition. In at least one embodiment of particular interest, from 50 to 100% by weight, more particularly from 70 to 100% by weight, even more particularly from 90 to 100% by weight of the total carrier oil is provided by one or more non-volatile oils, the non-volatile oils preferably being selected from hydrocarbon oils , triglyceride oils, and silicone oils. In use, the carrier oil helps to impart a smooth and moisturizing skin feel.

As used herein reference to "carrier oil" is exclusive of fragrance and the class of plant derived materials commonly known as essential oils.

The solid cleansing surfactant is a synthetic detersive surfactant, i.e., syndet, that has a melting point above 25°C, preferably above 35°C, more preferably above 40°C. In one or more embodiments, cleansing surfactant having a melting point above 50°C is of particular intent. Commonly, the solid cleansing surfactant includes one or more surfactants selected from anionic surfactant, zwitterionic surfactant, amphoteric surfactant and mixtures thereof.

In the surfactant descriptions that follow, carbon contents ought to be understood as modified by the word "about".

The anionic surfactant may be, for example, an aliphatic sulfonate, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGES); or an aromatic sulfonate such as alkyl benzene sulfonate. The anionic surfactant may also be an alkyl sulfate (e.g., C₈-C₂₂ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 22 carbons, preferably from 8 to 18 carbons, more preferably from 12 to 18 carbons; n has an average value of greater than 1.0, preferably between 2 and 3; and M is a solubilizing cation such as, for example, sodium, potassium, ammonium or substituted ammonium. Ammonium and sodium laurel ether sulfates are preferred. The anionic surfactant may also be alkyl sulfosuccinates (including mono- and dialkyl, e.g., C₈-C₂₂ sulfosuccinates); alkyl and acyl taurates, alkyl and acyl sarcosinates, sulfoacetates, C₈-C₂₂ alkyl phosphates and phosphates, alkyl phosphate esters and alkoxyl alkyl phosphate esters, acyl lactates, C₈-C₂₂ monoalkyl succinates and maleates, sulphoacetates, acyl isethionates, and acyl glycinates.

Sulfosuccinates may be monoalkyl sulfosuccinates having the formula:

R¹O₂CCH₂(SO₃M)CO₂M ;

and amido-MEA sulfosuccinates of the formula:

R¹CONHCH₂CH₂O₂CCH₂CH(SO₃M)CO₂M ;

wherein in such formulas R¹ ranges from C₈-C₂₂ alkyl and M is a solubilizing cation as described above; or amido-MIPA sulfosuccinates of formula:

RCONH(CH₂)CH(CH₃)(SO₃M)CO₂M

wherein R and M are as described above.

Also included are the alkoxylated citrate sulfosuccinates; and alkoxylated sulfosuccinates such as the following:

R-O-(CH₂CH₂O)ₘ-C(O)-CH₂CH(SO₃M)CO₂M)

wherein m=1 to 20; and R and M are as described above.

Sarcosinates are generally indicated by the formula R²CON(CH₃)CH₂CO₂M, wherein R² ranges from C₈ to C₂₀ alkyl and M is a solubilizing cation.

Taurates are generally identified by formula:

R²CONR³CH₂CH₂ SO₃M

wherein R² is described above, R³ ranges from C₁-C₄ alkyl and M is a solubilizing cation.

Another class of anionic surfactants are carboxylates such as follows:

R²(CH₂CH₂O)ₚM

wherein R² is as described above, p is 0 to 20, and M is a solubilizing cation. Yet another carboxylate which can be used is amido alkyl polypeptide carboxylates such as, for example, is available from Seppic under the trademark Monteine^{®}.

Yet another surfactant which may be used herein are the acyl isethionates, in particular, the C₆-C₂₂ acyl isethionates. These esters are typically prepared by reaction between alkali metal isethionate with mixed aliphatic fatty acids having from 6 to 22 carbon atoms and an iodine value of less than 20, preferably at least 75% of the mixed fatty acids have from 12 to 18 carbon atoms. The acyl isethionate may be an alkoxylated isethionate such as is described in Ilardi et al., U.S. Pat. No. 5,393,466, incorporated herein by reference.

Acyl glycinates are representative of yet another class of suitable anionic surfactants. The acyl glycinates are generally represented by the formula:

R²CONHCH₂COOM

where R² and M are as described above. Suitable acyl glycinates include for example, sodium cocoyl glycinate, sodium stearoyl glycinate, sodium lauroyl glycinate, sodium lauroyl sarcosinate, and potassium cocoyl glycinate.

In general, the anionic component will comprise from 0 to 100% by weight, preferably from 30 to 100% by weight, most preferably from 40 to 90% by weight, or higher, of the total solid cleansing surfactant.

Zwitterionic surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents has from 8 to 22 carbon atoms and one contains an anionic group, e.g., carboxy, sulfonate, sulfate, phosphate, or phosphonate. A general formula for many of these compounds is: 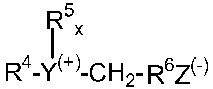 wherein R⁴ contains an alkyl, alkenyl, or hydroxy alkyl radical of from 8 to 22 carbon atoms, from 0 to about 10 ethylene oxide moieties and from 0 to about 1 glyceryl moiety; Y is selected from the group consisting of nitrogen, sulfur and phosphorous atoms; R⁵ is an alkyl or monohydroxyalkyl group of from 1 to 3 carbon atoms; x is 1 when Y is a sulfur atom, and 2 when Y is a nitrogen or phosphorus atom; R⁶ is an alkylene or hydroxyalkylene of from 1 to 4 carbon atoms and Z is a radical selected from the group consisting of carboxylate, sulfonate, sulfate, phosphonate, and phosphate groups.

Amphoteric detergents which may be used in this invention include at least one acid group. This may be a carboxylic or a sulphonic acid group. They include quaternary nitrogen and therefore are quaternary amido acids. They should generally include an alkyl or alkenyl group of 7 to 18 carbon atoms. They will usually comply with an overall structural formula: 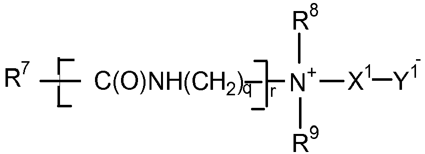 where R⁷ is alkyl or alkenyl of 7 to 18 carbon atoms; R⁸ and R⁹ are each independently alkyl, hydroxyalkyl or carboxyalkyl of 1 to 3 carbon atoms; q is 2 to 4; r is 0 to 1; X¹ is alkylene of 1 to 3 carbon atoms optionally substituted with hydroxyl, and Y¹ is --CO₂-- or -SO₃--.

Suitable amphoteric detergents within the above general formula include simple betaines of formula: 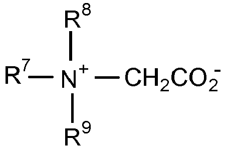 and amido betaines of formula: 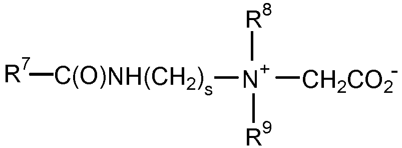 where s is 2 or 3. In both formulae R⁷, R⁸ and R⁹ are as previously described R⁷ may, in particular, be a mixture of alkyl groups derived from coconut so that at least half, preferably at least three quarters of the groups R⁷ have 10 to 14 carbon atoms. Preferably, R⁸ and R⁹ are methyl.

A further possibility is that the amphoteric detergent is a sulphobetaine of formula 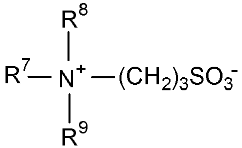 or 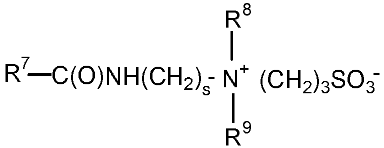 where s is as previously described, and preferably is 2, or variants of these in which -(CH₂)₃ SO₃⁻ is replaced by -CH₂C(OH)HCH₂SO₃⁻. In these formulae R⁷, R⁸ and R⁹ are as discussed previously. Amphoacetates and diamphoacetates are also intended to be covered in possible zwitterionic and/or amphoteric compounds which may be used.

Together, zwitterionic and amphoteric surfactant may comprise from 0 to 100% by weight of the total solid cleansing surfactant and, when present, will preferably comprise from 10 to 60% by weight of the total solid cleansing surfactant. In one or more preferred embodiments, together, anionic surfactant and amphoteric surfactant comprise 80 to 100%, more particularly 90 to 100% by weight of the total solid cleansing surfactant.

The composition may optionally, though less commonly, comprise other types of solid cleansing surfactant, e.g., nonionic and cationic surfactant. If present the total amount thereof is typically from 0.1 to 40% by weight and, more particularly, from 0.5 to 10% by weight of the total solid cleansing surfactant.

In one or more embodiments compositions having relatively high levels of solid cleansing surfactant, for example from 35 to 65% by weight and, more particularly, from 40 to 60% by weight, based on the total weight of the composition, are of interest. In at least one embodiment, the composition comprises from 35 to 55% by weight of solid cleansing surfactant, based on the total weight of the composition.

The solid cleansing surfactant is present in the subject composition in the form of dispersed particles. As used herein, all references to "size" and "size distribution" of solid cleansing surfactant particles are to the particles as primary particles; where the particles are in aggregated form, these parameters are given in reference to the particles that make-up the aggregate, as opposed to the aggregate made up of the particles. The size of the solid cleansing surfactant particles is within a range from 0.5 to 2000µm, wherein at least 50% by weight, preferably at least 60% by weight, more preferably at least 65% by weight of the particles are less than 200µm in size and at least 30% by weight, preferably at least 40% by weight, most preferably at least 50% by weight of the particles are less than 150µm in size. Preferably no more than 45% by weight, more preferably no more than 30% by weight of the solid surfactant particles are great than 500µm in size. In reference to a particle, size refers to the the particle's largest diameter, as a primary particle.

In addition to impacting product stability, viscosity, and appearance, the size and size distribution of the solid cleansing surfactant particles can also affect in-use properties such as speed to lather and product "feel". In at least one embodiment, a desirable combination of properties is provided by the particles of solid cleansing surfactant having a size distribution wherein at least 40% by weight, preferably at least 60% by weight, most preferably at least 80% by weight of the particles are of particle size in the range of 1 to 200 microns and less than 20% by weight, preferably less than 15% by weight, most preferably less than 10% by weight of the particles are of particle size larger than 500 microns, with the maximum size of such particles preferably being no more than 2000 microns and, more preferably, no more than 1000 microns. In one or more embodiments. no more than 30% by weight, preferably no more than 15% by weight of the of the solid cleansing surfactant are particles greater than 300 microns in size.

Particle sizes greater than 200 microns can be perceived as "gritty"; accordingly, in one or more embodiments it is preferred that at least 95% by weight, more preferably at least 97% by weight, even more preferably at least 99% by weight of the particles of solid surfactant range are of size of not more than 200 microns. Conversely particle sizes of less than 5 microns can require specialized handling. In one or more embodiments it is preferred that at least 97% by weight, more preferably at least 99% by weight, even more preferably essentially all, of the solid cleansing surfactant particles are of size with a range of 5 to 200µm, more particularly 5 to 150 microns, even more particularly from 10 to 120 microns. In the subject compositions, aggregation of the solid cleansing surfactant results in the formation of a loose network structure in which the primary surfactant particles form aggregates or clusters that typically contain on the order of 2 to 20 particles.

The solid cleansing surfactants suitable for use herein can be supplied as free flowing powders, flakes, granules or as a pre-dispersion of the solid surfactant particles in carrier oil. To minimize the handling issues associated with the use of fine powders, especially on a large or production scale, it is especially desirable to employ the powders as a pre-dispersion of solid surfactant in carrier oil, preferably with a particle size/size distribution as described above. Solid cleansing surfactant obtained as granules or flakes are preferably premixed with carrier oil followed by high shear mixing to grind or mill the solid granules or flakes to the desired size/size distribution.

Together, the carrier oil and solid cleansing surfactant preferably comprise from 60 to 100% by weight, more particularly from 75 to 98% by weight, of the subject compositions.

The primary particles of solid cleansing surfactant may be employed in a pre-aggregated state or may be aggregated in situ. One means of preparing pre-aggregated surfactant particles is to use production techniques in which an aqueous surfactant solution is sprayed and atomized to micron size droplets into a hot air drying chamber in which water is evaporated relatively quickly to create free flowing particles. Owing to the presence of moisture, these dried surfactant particles tend to stick together thereby forming aggregates. Pre-formed aggregated surfactant particles are available commercially and include, for example, sodium N-cocoyl glycinate powder available from Ajinomoto under the trade name Amilite^{®} GCS-11 and cocoamidopropylbetaine powder available from Degussa under the trade name Tego^{®}Betaine CKD.

Aggregation of the primary particles of solid surfactant can also be accomplished in situ by the inclusion of a small amount of water, i.e., an amount of from 0.05 to 5% by weight, preferably 0.1 to 4% by weight, more preferably 0.2 to 3% by weight, based on the total weight of the composition. Excess amounts of water can result in a product that is lumpy and/or that has an undesirably high viscosity. Water is present in an amount less than 5% by weight and, more particularly, less than 4% by weight, based on the total weight of the composition. Conversely, insufficient water can fail to provide desirable aggregation. In one or more embodiments of particular interest, water is present in an amount of 0.5 to 3% by weight, based on the total weight of the composition. In other embodiments of interest the compositions of this invention comprise from 0.2 to 2% by weight of water.

Water soluble polymers are an optional ingredient that, in one or more embodiments, are highly preferred to be included in the composition of the invention. Water soluble polymers commonly have a solubility in water of at least 1% at 25°C. These polymers can be cationic, anionic, amphoteric and/or nonionic types. They are known to enhance in-use and after-use skin/hair sensory feels, and to enhance lather creaminess, lather lubricity and lather stability. Water soluble cationic polymers are also known to enhance oil deposition. To provide enhanced in-use and after-use benefits from the composition, polymers that are readily soluble upon contact with water are particularly useful for the oil continuous composition of this invention.

The amount of the water soluble polymers, when present, is typically 0.005 to 5% by weight, more particularly 0.01 to 3% by weight, based on the total weight of the composition. The polymers can be added into the composition of this invention as fine powder or, subject to the total water content parameters noted above, as aqueous solutions. The water soluble polymer is generally of relatively high molecular weight.

The average molecular weight of polymeric materials such as water-soluble polymers can be determined by any of a variety of different procedures, as appropriate to the particular type of polymer. For example, rheological measurements can be used to obtain viscosity average molecular weights and gel permeation chromatography or light scatter method can be used to obtain number average molecular weights. The average molecular weights reported by suppliers of such polymers is typically higher than 10,000 Daltons, and frequently is at least 50,000 Daltons and, more particularly, at least 100,000 Daltons, with the means for measuring same and basis on which average molecular weight is reported, for example, number average molecular weight, viscosity average molecular weight, or weight average molecular weight, being subject to variation. Commonly, the polymers of interest have a number average molecular weight that is at least 50,000 Daltons and, more particularly is at least 100,000 Daltons, and frequently is less than 5,000,000 Daltons.

Examples of water soluble polymers include high molecular weight polyethylene glycols such as Polyox^{®} WSR-205 (PEG 14M), Polyox^{®} WSR-N-60K (PEG 45M), and Polyox^{®} WSR-301 (PEG 90M); the carbohydrate gums such as cellulose gum. hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, and xanthan gum; modified starch granules and pregelatinized cold water soluble starch; cationic polymer such as modified polysaccharides including cationic guar available from Rhodia under the trade name Jaguar^{®}; cationic modified cellulose such as UCARE Polymer JR 30 or JR 40 from Amerchol; N-Hance^{®} 3000, N-Hance^{®} 3196, N-Hance^{®} GPX 215 or N-Hance^{®} GPX 196 from Hercules; synthetic cationic polymers such as Merquat^{®} 100, Merquat^{®} 280, Merquat^{®} 281 and Merquat^{®} 550 sold by Nalco. The water soluble polymers may be used individually or as combinations of two or more polymers from the same or different classes. High molecular weight polyethylene glycols Polyox^{®} WSR-301 (PEG 90M) and Polyox^{®} WSR-N-60K (PEG 45M) and guar derivatives such as Jaguar^{®} S, Jaguar^{®} C17, and Jaguar^{®} C13, and synthetic cationic polymers such as Merquat^{®} 100 are particularly desired.

Optionally, the compositions of this invention may further comprise one or more additional ingredients. Non-limiting examples of such additional ingredients are, for example, colorants, pigments, opacifiers, fragrance (whether encapsulated or present as free-fragrance), emotive oils, vitamins and vitamin derivatives, abrasives, optical agents (including for example, reflective particles and interference pigments), pH adjusters, plant extracts, essential oils, preservatives, antioxidants, antimicrobials, viscosity modifiers, humectants, beard wetting agents, sensory agents, fatty acid soap, and skin and/or hair benefit agents (e.g., aloe, allantoin, panthenol, alpha-hydroxy acids, phospholipids, botanical oils, and amino acids to name a few). The selection and amount of any individual additional ingredient depends upon factors that include the particular ingredient, the properties desired, and the intended use of the composition in which it is employed. For example, fragrance is typically employed in an amount of 0.1 to 3.0% by weight of the composition, or higher. For many compositions, the total amount of such additional ingredients is 0.01 to 30% by weight, more particularly, 0.1 to 15% by weight, even more particularly, 1 to 10% by weight, based on the total weight of the composition. In one or more embodiments, the total amount of such additional optional ingredients is 0.5 to 5% by weight. Other ingredients, for example fatty acid soap, may be present at levels up to 10% by weight, based on the total weight of the composition.

The compositions are conveniently prepared by dispersing the solid cleansing surfactant in the carrier oil and mixing the resulting dispersion under shear, preferably under high shear, adding water, as needed, to achieve particle aggregation. Mixing is desirably carried out until the desired size and size distribution of the solid cleansing surfactant particles is achieved. Alternatively, pre-aggregated surfactant particles may be employed as a starting material and mixing conditions adjusted accordingly. Mixing can be carried out with little or no applied heat, with mixing at temperatures that nominally (i.e., without considering heat imparted to the mixture by shear) are ambient being preferred. Commonly such temperatures are in the range of 20 to 25°C. Additional components may be added prior to, during and/or after shear mixing, as appropriate; for example, volatile or shear sensitive components are typically added toward the completion of and/or after shear mixing to minimize their degradation or loss.

The compositions may be formulated as any of a variety of different personal care products including, for example, body washes, hand cleansers, facial cleansers, make-up removers, shaving compositions, and the like. In use, the compositions are compositions are diluted with water, typically in a weight ratio of composition to water of from 1 to 20, more particularly from 1 to 10, and worked into a lather before, during or after application, optionally with the aid of a sponge, pouf, cloth, brush or the like. After being used for their intended application, be it as a cleanser, shaving composition, make-up remover, or the like, the residual diluted product is removed, commonly by rinsing with water.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above. In specifying any range of concentration or amount, any particular upper concentration or amount can be associated with any particular lower concentration or amount. Reported ranges are inclusive of their endpoints. Unless otherwise indicated, melting points referenced herein are at ambient pressure, i.e. 1 atmosphere.

Except in the operating and comparative examples, or where otherwise explicitly indicated, all numbers in this description indicating amounts, parts, percentages, ratios and proportions of material, and conditions of reaction ought to be understood as modified by the word "about".

The following examples will more fully illustrate the embodiments of this invention. The Examples are not intended to limit the scope of the invention in any manner. All parts, percentages and proportions referred to herein and in the appended claims are by weight unless otherwise indicated.

### EXAMPLES

### Examples 1 to 6

This series of Examples shows the effect of aggregates of solid surfactant particles on the stability of oil continuous compositions containing dispersed surfactant particles.

Simple anhydrous surfactant dispersions containing one of two different solid surfactants, i.e., sodium lauroyl isethionate (SLI) powder (Yongan Daily Chemical Co.) or sodium cocoyl glycinate (SCG) particles (Amilite^{®} GCS-11 from Ajinomoto) were prepared by adding about 0.2g of the solid surfactant (i.e., SLI powder or SCG particles) to 9.8g mineral oil (Penreco Drakeol^{®} 7 white mineral oil) in a small glass vial and mixing first with a spatula for about 30 seconds. The dispersion was then mixed uniformly using a vertex mixer (MaxiMix™ II from Thermolyne) for about 1 minute. After this machine mixing, the particle in oil dispersion was placed on a glass slide and a picture was taken using an optical microscope at 50 times magnification. The particle size and particle size distribution of the dispersions was evaluated.

In the SLI-containing dispersion, the powder had an irregular particle shape and a size of from several micrometers up to about 200 micrometers; the particles were separated, well-dispersed, and non-aggregated. In contrast, in the SCG-containing dispersion most of the SCG particles were in the form of irregularly shaped particle aggregates about 30 to 300 micrometers in length, with the particles making up the aggregates generally being spherically shaped and of a size of from about 10 to about 80 micrometers.

The SLI and SCG solid surfactants were also used to prepare a series of cleansing compositions as described in Table 1. The Table 1 cleansing compositions were prepared by first adding the oil to a glass jar equipped with a 3-blade mixer (IKA). The water soluble polymer powders (i.e. Polyox™ WSR-301 PEG 90M or Jaguar^{®} S guar gum) and perfume were added and mixed with the oil. The solid surfactant was then gradually added to the oil over a period of 5 to 10 minutes while mixing at room temperature (20 to 25°C). After adding all the solid surfactant, the mixture was mixed at high speed (1000 to 1500 rpm) for another 3 to 6 minutes to uniformly disperse the solid surfactant particles in the oil. For Examples 4, 5 and 6, deionized water was added slowly to the mixer for 1 to 3 minutes while mixing at 1000 to 1500 rpm.

After preparation, these samples were aged at room temperature for 7 days and then evaluated for product stability and viscosity, the results of which are reported in Table 1.

Stability was determined as a function of oil separation, employing an approximately 100g sample that was introduced to a 4oz (118ml) cylindrical glass jar having an inner diameter of 1.8 inches (4.6cm). If a sample was not stable, disruption of the oil phase and separation of the oil/surfactant dispersion occurred, resulting in a layer of oil on top of the sample. The height of the oil layer was measured and divided by the total height of the sample and reported as the "% oil layer separation". A 0% oil layer separation means the sample was stable without any visible phase separation of surfactant particle from the oil.

Viscosity of the aged sample was measured using a standard AR-G2 stress-controlled rheometer from Texas instruments (or equivalent) and carrying out steady shear rate sweep measurements from 0.01 to 100s⁻¹, using a 40mm cone and plate geometry, with a cone angle of 2° and a sample gap of 61 microns and collecting three sample points per decade, with all the measurements being done at constant temperature of 23°C.

**Table 1**

| | **Example** | | | | | |
|---|---|---|---|---|---|---|
| **Ingredient** (Wt.%) | 1 | 2 | 3 (Comp.) | 4 | 5 | 6 |
| White Mineral Oil (Drakeol^{®} 7 Lt. Min. Oil, ex.Penreco)¹ | 48.7 | 68.5 | 48.5 | 45.0 | 46.2 | 48.0 |
| Sodium Lauroyl Isethionate Powder (ex. Yongan Daily Chemical Co.) | 30.0 | | 50.0 | 50.0 | 50.0 | 50.0 |
| Sodium Cocoyl Glycinate Particles (Amilite^{®} GCS-11, ex. Ajinomoto) | 20.0 | 30.0 | | | | |
| Water | 0 | 0 | 0 | 3.5 | 2.5 | 0.5 |
| PEG-90M (Polyx™ WSR-301 Water Soluble Resin ex. Dow Chemical)² | 0.3 | | - | - | 0.3 | 0.3 |
| Guar Gum; CAS No. 9000-30-0 (Jaguar^{®} S Ex. Rhodia) | -- | 0.5 | 0.5 | 0.5 | - | 0.2 |
| Perfume | 1.0 | 1.0 | 1.0 | 1.0 | 1.0 | 1.0 |
| | | | | | | |
| % oil layer separation | 0% | 0% | 27.4% | 0% | 0% | 8.2% |
| | | | | | | |
| Viscosity at 0.01 sec⁻¹ (kg•m⁻¹ s⁻¹ | 18100 | 52310 | 291 | 2180 0 | 1826 | 493 |
| Viscosity at 1 sec⁻¹ (kg•m⁻¹ s⁻¹) | 88.8 | 163.7 | 7.5 | 60.0 | 16.2 | 8.6 |

| | | | | | | |
|---|---|---|---|---|---|---|
| 1 Reported by the Supplier as having a typical viscosity (CST@40°C; ASTM D445) of 12.59. 2 Reported by the Supplier as having an approximate molecular weight of 4,000,000 and viscosity (cPs) of 1650-5500.. | | | | | | |

As demonstrated by Examples 4 to 6, the addition of water to compositions containing the SLI power improved composition stability and increased viscosity at both 0.01 and 1 sec⁻¹. Additionally, as demonstrated by Examples 1 and 2, compositions containing no added water and the pre-aggregated SCG surfactant were stable. In the case of Example 1, stability was achieved notwithstanding the addition of the non-aggregated SLI powder.

Without wishing to be bound to theory, it is believed that the addition of water induces interaction of individual surfactant particles in the oil phase to form surfactant particle aggregates which can increase the composition viscosity and promote oil phase stability.

Using the mixing method described above, the composition of Example 4 was diluted to an SLI content of 2wt.% by the addition of additional mineral oil (Penreco Drakeol^{®} 7 white mineral oil). The diluted Example 4 composition was examined using optical microscopy following the procedure described above; an examination of the resulting micrograph revealed that the surfactant particles are generally aggregated, thus, the composition was found to exhibit water-induced surfactant aggregation.

### Examples 7 to 13

Oil continuous cleansing compositions were prepared according to the formulations described in Table 2 using different oils and, as the inducers of particle aggregation, various water-containing ingredients.

The neutralized fatty acid dispersions reported as ingredients were prepared by combining 47.4 parts of the indicated oil, 21.6 parts of lauric acid, 10.8 parts of myristic acid and 3.6 parts stearic acid in a vessel equipped with a 3-blade overhead mixer (IKA) and heating the resulting oil/acid mixture to 70-75°C, with stirring; when all the fatty acids had dissolved (as indicated by the formation of a clear solution); 16.6 parts of a solution of 45 wt% KOH in water was slowly added to the fatty/acid oil solution over a period of 5 to 10 minutes to neutralize the fatty acids; after mixing for an additional 5 minutes at 70°C, the mixture was cooled to ambient temperature, yielding a dispersion of fine soap crystals in oil.

The Table 2 formulations were prepared by first adding the indicated oil component to a glass jar equipped with an overhead 3-blade mixer. The water soluble polymer powders (i.e. Polyox™ WSR-301(PEG-90M) or Jaguar^{®} S guar gum) and perfume were added and mixed with the oil.

The surfactant was then gradually added over a period of 5 to 10 minutes while mixing at room temperature (20 to 25°C) to uniformly disperse the solid surfactant particles in the oil. After addition of the surfactant was complete, the remaining ingredients were added and the mixture was mixed at high speed (1000 to 1500 rpm) for another 3 to 6 minutes. The formulations were aged at room temperature for seven days and then evaluated for product stability and viscosity, the results of which are reported in Table 2.

**Table 2**

| | **7** | **8** | **9** | **10 (Comp)** | **11 (Comp)** | **12 (Comp)** | **13 (Comp)** |
|---|---|---|---|---|---|---|---|
| **Ingredient (wt.%)** | | | | | | | |
| White Mineral oil (Drakeol^{®} 7 Lt. Min. Oil, ex Penreco) | 45.70 | | | 48.50 | - | - | - |
| Mineral Oil (Hydrobrite^{®} 1000, ex Sonneborn) | | 50.31 | 48.80 | - | - | - | 58.80 |
| Soybean oil | | | | - | 58.85 | - | - |
| Silicone Fluid (Dimethicone; 350 cps) | | | | | | 68.85 | |
| Na lauroyl isethionate powder (ex. Yongan Daily Chemical Co.) | 50.00 | 38.85 | 40.00 | 50.00 | 40.00 | 30.00 | 40.00 |
| Guar Gum; CAS No. 9000-30-0 (Jaquar^{®} S ex. Rhodia) | | | | 0.50 | - | - | - |
| PEG-90M (Polyox™ WSR-301 Water Soluble Resin ex. Dow Chemical) | 0.30 | 0.15 | 0.2 | - | 0.15 | 0.15 | 0.20 |
| Perfume | 1.00 | 0.99 | 1.00 | 1.00 | 1.00 | 1.00 | 1.00 |
| Steol® 170 (70% Na laureth sulfate ex. Stepan) | | 9.70 | | | | | |
| Tego® Betain CKD powder ex. Evonik | | | 5.00 | | | | |
| Tego® Betain F (28% Na cocoyl aminopropyl betaine) | 3.00 | | | | | | |
| DC 1728 silicon emulsion (60% solid from Dow Corning) | | | 5.00 | | | | |
| Total free water content (From water-containing ingredients) | 2.2 | 2.9 | 2.0 | 0.0 | 0.0 | 0.0 | 0.0 |
| % clear oil layer separation | 0% | 0% | 0% | 27.4% | 25.8% | 23.3% | 12.2% |
| Viscosity at 0.01 sec⁻¹ (kg•m⁻¹) | 128900 | 3599 | 2327 | 291 | 11 | 9 | 22 |
| Viscosity at 1 sec⁻¹ (kg•m⁻¹) | 1048 | 128 | 202 | 7.5 | 1 | 2 | 3 |

These examples show that the various water-containing ingredients other than water alone can be used to enhance product viscosity to prevent or minimize the suspended surfactant particles from separating from the oil.

## Claims

1. A foamable, fluid personal care composition that comprises a continuous oil phase comprising at least one carrier oil and dispersed aggregated particles of solid cleansing surfactant, wherein the composition comprises:
i. 35 to 90 % by weight, based on the total weight of the composition, of carrier oil;
ii. 10 to 65% by weight, based on the total weight of the composition of solid cleansing surfactant wherein the solid surfactant is present in the form of particles of size within a range of from 0.5 to 2000µm wherein at least 50% of the particles are less than 200µm in size and at least 30% of the particles are less than 150µm in size.; and
iii. 0.05 to 5% by weight, based on the total weight of the composition, of water.

2. The composition according to claim 1 wherein water is present in an amount of from 0.2 to 4% by weight, based on the total weight of the composition.

3. The composition according to claim 1 wherein the carrier oil includes one or more oils selected from hydrocarbon oils, ether oils, ester oils, fatty alcohols, and silicone oils.

4. The composition according to claim 1 wherein from 90 to 100% by weight of the carrier oil is one or more oils selected from non-volatile hydrocarbon, triglyceride, and silicone oils.

5. The composition according to claim 1 wherein the carrier oil comprises at least 95% by weight of non-volatile oil.

6. The composition according to claim 1 wherein the carrier oil is present in an amount of from 40 to 80% by weight, based on the total weight of the composition.

7. The composition according to claim 1 wherein the solid surfactant is present in an amount of 35 to 60% by weight, based on the total weight of the composition.

8. The composition according to claim 1 wherein at least a portion of the solid surfactant is in the form of pre-aggregated particles.

9. The composition according to claim 1 wherein the solid surfactant is aggregated in situ.

10. The composition according to claim 1 having a viscosity in the range of from 300 to 50,000kgm⁻¹s⁻¹ at 0.01 rps and 23°C.

11. The composition according to claim 1 wherein water is present in an amount of from 0.5 to 3% by weight, based on the total weight of the composition.

12. A method for preparing a composition as described in claim 1 wherein the solid surfactant is dispersed with the carrier oil and mixed under shear adding water, as needed, to achieve particle agglomeration.

13. The method as described in claim 12 wherein the solid surfactant and carrier oil are mixed under shear at temperature in the range of 20 to 25°C.

## Patentansprüche

1. Schäumbare, flüssige Körperpflegezusammensetzung, die eine kontinuierliche Ölphase umfasst, die mindestens ein Trägeröl und dispergierte aggregierte Partikel von festem Reinigungstensid umfasst, wobei die Zusammensetzung umfasst:
i. 35 bis 90 Gewichts-%, bezogen auf das Gesamtgewicht der Zusammensetzung, an Trägeröl,
ii. 10 bis 65 Gewichts-%, bezogen auf das Gesamtgewicht der Zusammensetzung, an festem Reinigungstensid, wobei das feste Tensid in Form von Partikeln einer Größe innerhalb eines Bereichs von 0,5 bis 2000 µm vorliegt, wobei mindestens 50% der Partikel weniger als 200 µm groß sind und mindestens 30% der Partikel weniger als 150 µm groß sind, und
iii. 0,05 bis 5 Gewichts-%, bezogen auf das Gesamtgewicht der Zusammensetzung, an Wasser.

2. Zusammensetzung nach Anspruch 1, wobei Wasser in einer Menge von 0,2 bis 4 Gewichts-%, bezogen auf das Gesamtgewicht der Zusammensetzung, vorliegt.

3. Zusammensetzung nach Anspruch 1, wobei das Trägeröl ein oder mehrere Öle einbezieht, die unter Kohlenwasserstoffölen, Etherölen, Esterölen, Fettalkoholen und Silikonölen ausgewählt sind.

4. Zusammensetzung nach Anspruch 1, wobei 90 bis 100 Gewichts-% des Trägeröls durch ein oder mehrere Öle dargestellt wird, die unter nichtflüchtigem Kohlenwasserstoff, Triglycerid und Silikonölen ausgewählt sind.

5. Zusammensetzung nach Anspruch 1, wobei das Trägeröl mindestens 95 Gewichts-% nichtflüchtiges Öl umfasst.

6. Zusammensetzung nach Anspruch 1, wobei das Trägeröl in einer Menge von 40 bis 80 Gewichts-%, bezogen auf das Gesamtgewicht der Zusammensetzung, vorliegt.

7. Zusammensetzung nach Anspruch 1, wobei das feste Tensid in einer Menge von 35 bis 60 Gewichts-%, bezogen auf das Gesamtgewicht der Zusammensetzung, vorliegt.

8. Zusammensetzung nach Anspruch 1, wobei mindestens ein Teil des festen Tensids in Form von voraggregierten Partikeln vorliegt.

9. Zusammensetzung nach Anspruch 1, wobei das feste Tensid *in situ* aggregiert ist.

10. Zusammensetzung nach Anspruch 1, die eine Viskosität in dem Bereich von 300 bis 50.000 kgm⁻¹s⁻¹ bei 0,01 U/s und 23°C aufweist.

11. Zusammensetzung nach Anspruch 1, wobei Wasser in einer Menge von 0,5 bis 3 Gewichts-%, bezogen auf das Gesamtgewicht der Zusammensetzung, vorliegt.

12. Verfahren zur Herstellung einer Zusammensetzung, wie im Anspruch 1 beschrieben, wobei das feste Tensid mit dem Trägeröl dispergiert und unter Scherung, bei Bedarf unter Zugabe von Wasser, gemischt wird, um die Partikelagglomeration zu erreichen.

13. Verfahren, wie im Anspruch 12 beschrieben, wobei das feste Tensid und das Trägeröl bei einer Temperatur in dem Bereich von 20 bis 25°C unter Scherung gemischt werden.

## Revendications

1. Composition fluide, apte à mousser pour le soin de la personne qui comprend une phase d'huile continue comprenant au moins une huile de support et des particules agrégées dispersées de tensioactif nettoyant solide, dans laquelle la composition comprend
i. de 35 à 90 % en masse, rapporté à la masse totale de la composition, d'huile de support ;
ii. de 10 à 65 % en masse, rapporté à la masse totale de la composition de tensioactif nettoyant solide dans laquelle le tensioactif solide est présent dans la forme de particules de dimension dans un intervalle de 0,5 à 2 000 µm, dans laquelle au moins 50 % des particules sont de taille inférieure à 200 µm et au moins 30 % des particules sont de taille inférieure à 150 µm ; et
iii. de 0,05 à 5 % en masse, rapporté à la masse totale de la composition, d'eau.

2. Composition selon la revendication 1, dans laquelle de l'eau est présente dans une quantité de 0,2 à 4 % en masse, rapporté à la masse totale de la composition.

3. Composition selon la revendication 1, dans laquelle l'huile de support comprend une ou plusieurs huiles choisies parmi des huiles hydrocarbonées, des huiles d'éthers, des huiles d'esters, des alcools gras, et des huiles de silicone.

4. Composition selon la revendication 1, dans laquelle de 90 à 100 % en masse de l'huile de support sont une ou plusieurs huiles choisies parmi des huiles hydrocarbonées, de triglycérides et de silicones non volatiles.

5. Composition selon la revendication 1, dans laquelle l'huile de support comprend au moins 95 % en masse d'huile non volatile.

6. Composition selon la revendication 1, dans laquelle l'huile de support est présente dans une quantité de 40 à 80 % en masse, rapporté à la masse totale de la composition.

7. Composition selon la revendication 1, dans laquelle le tensioactif solide est présent dans une quantité de 35 à 90 % en masse, rapporté à la masse totale de la composition.

8. Composition selon la revendication 1, dans laquelle au moins une portion du tensioactif solide est dans la forme de particules pré-agrégées.

9. Composition selon la revendication 1, dans laquelle le tensioactif solide est agrégé in situ.

10. Composition selon la revendication 1 ayant une viscosité dans l'intervalle de 300 à 50 000 kgm⁻¹s⁻¹ à 0,01 tr/s et à 23°C.

11. Composition selon la revendication 1, dans laquelle de l'eau est présente dans une quantité de 0,5 à 3 % en masse, rapporté à la masse totale de la composition.

12. Procédé de préparation d'une composition selon la revendication 1, dans laquelle le tensioactif solide est dispersé avec l'huile de support et mélangé sous cisaillement en ajoutant de l'eau, si nécessaire, pour réaliser une agglomération de particules.

13. Procédé selon la revendication 12, dans lequel le tensioactif solide et l'huile de support sont mélangés sous cisaillement à une température dans l'intervalle de 20 à 25°C.
